# EUROPEAN PATENT APPLICATION

(11) **EP 1 892 243 A1**
(43) Date of publication of application: **27.02.2008**
(21) Application number: 06016132.0
(22) Date of filing: 02.08.2006
(51) Int. Cl.: C07D 417/12, C07D 275/04, A61K 31/427

(54) **Polymorphic forms of ziprasidone sulphate salts**

(71) Applicant: KRKA, tovarna zdravil, d.d., Novo mesto, 8501 Novo mesto (SI)
(72) Inventor: Ruzic, Milos, 3000 Celje (SI); Smrkolj, Matej, 1411 Izlake Zagorje ob Savi (SI); Pecavar, Anica, 8000 Novo mesto (SI); Stergar, Matej, 3000 Celje (SI); Makuc, Simon, 8000 Novo mesto (SI); Urska, Jursic, 8322 Stopice (SI); Zajc, Natalija, 8340 Crnomelj (SI)
(74) Representative: Becker Kurig Straus

(57) **Abstract**

The present invention relates to novel polymorphic forms of ziprazisidone hydrogen sulphate salts of formula I:
[Ziprasidone* H]⁺[X⁻]*Z H₂O
wherein
Ziprasidone is a compound of the formula 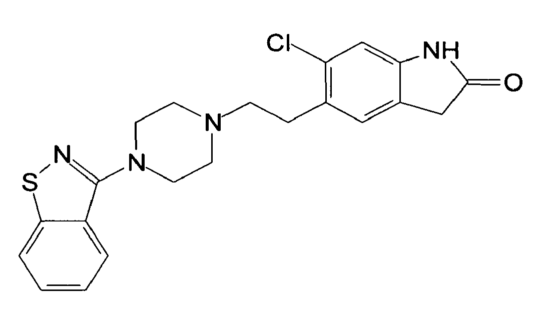 H is hydrogen
X is HSO₄⁻; and
Z is 0 to 30,
as well as to a process for their preparation and pharmaceutical formulations containing it. The present polymorphic forms of ziprazisidone sulphate salts are characterized by small average particle sizes and high solubility bestowing the compounds an improved bioavailability.

## Description

### Field of the invention

The present invention relates to novel polymorphic forms of ziprazisidone sulphate salts, as well as to a process for their preparation and pharmaceutical formulations containing it. The present polymorphic forms of ziprazisidone sulphate salts are characterized by small average particle sizes and high solubility bestowing the compounds an improved bioavilability.

### Background of the invention

Ziprasidone is the common name for 5-[2-[4-(1,2-benzisothiazol-3-yl)-1-piperazinyl]ethyl]-6-chloro-1, 3-dihydro-2H-indol-2-one, of the formula : 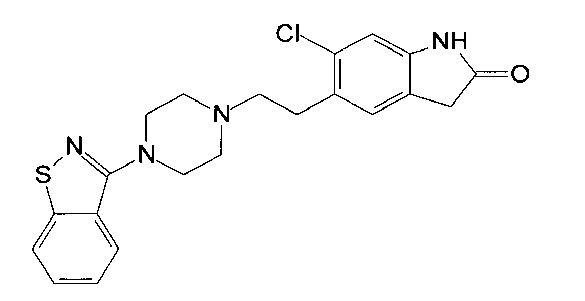 an active pharmaceutical ingredient having neuroleptic activity.

EP 0 281 309 decribes a process for the preparation of Ziprasidone by means of a coupling reaction performed in organic polar solvents, such as ethanol, N,N-dimethylformamide (DMF) and methylisobutylketone (MIBK). In Example 16 of EP 0 281 309-A1a process for preparing Ziprasidone hydrochloride in hemihydrated form is disclosed.

EP 0 584 903 discloses an alternative approach for the preparation of Ziprasidone, Ziprasidone salts including hydrochloride and methanesulfonate, wherein the coupling reaction is carried out in water in the presence of an excess of sodium carbonate as neutralizing agent.

According to EP 0 281 309, EP 0 584 903 and EP 1 029 861, pharmaceutically acceptable acid addition salts of the Ziprasidone may be prepared in a conventional manner by treating a solution or suspension of the Ziprasidone free base with about one chemical equivalent of a pharmaceutically acceptable acid. Conventional concentration and recrystallization techniques are employed in isolating the salts.

EP 0 918 772 and EP 0 904 273 refer to dihydrate and trihydrate salts of Ziprasidone mesylate which are reported to possess increased hygroscopic stability and increased aqueous solubility than the hydrochloride salts.

In WO2006/034964 a process of using organic polar solvents, such as acetonitrile, is disclosed for the preparation of Ziprasidone pharmaceutically acceptable acid addition salt, such as maleate or acetate.

In view of the prior art there still exists a need for an improved method for the preparation of Ziprasidone salts.

### Object of the invention

Accordingly, an object of the present invention resides in the provision of Ziprasidone salts with defined properties rendering them more suitable for the preparation of pharmaceutical compositions. Another object of the present invention resides in the provision of Ziprasidone salts, which may be better tolerated if ingested.

The present invention solves the above-mentioned problems by the provision of Ziprasidone sulphates with particular counter ions to the sulphate and specific amounts of crystal water. The present Ziprasidone sulphate salts may be obtained in high purity, have good stability and advantageous formulation properties particularly in comparison to the hydrochloride form of Ziprasidone. The present inventors have surprisingly found out that the present compounds provide improved properties with respect to solubility and particle size. The solubility of the compounds is on the one hand directly influenced by the content of crystal water and on the other hand by the low particle size. Said effects in turn influence tolerance and incorporation of said compounds upon ingestion, since smaller amounts of the drugs may be employed in respective pharmaceutical compositions exhibiting anyway the same or even improved bioavailability in comparison to compounds of the state of the art.

### Figures

In the figures are, where applicable, (a) table , (b) FT-IR spectra and (c) microscopic picture.

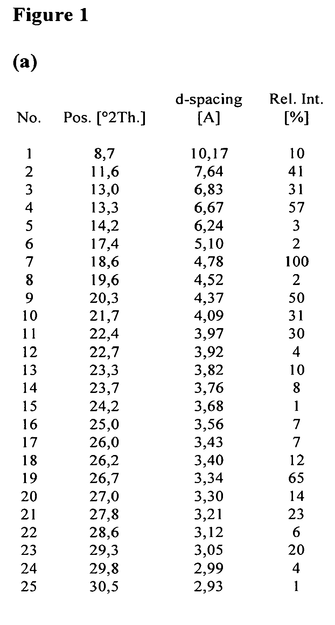

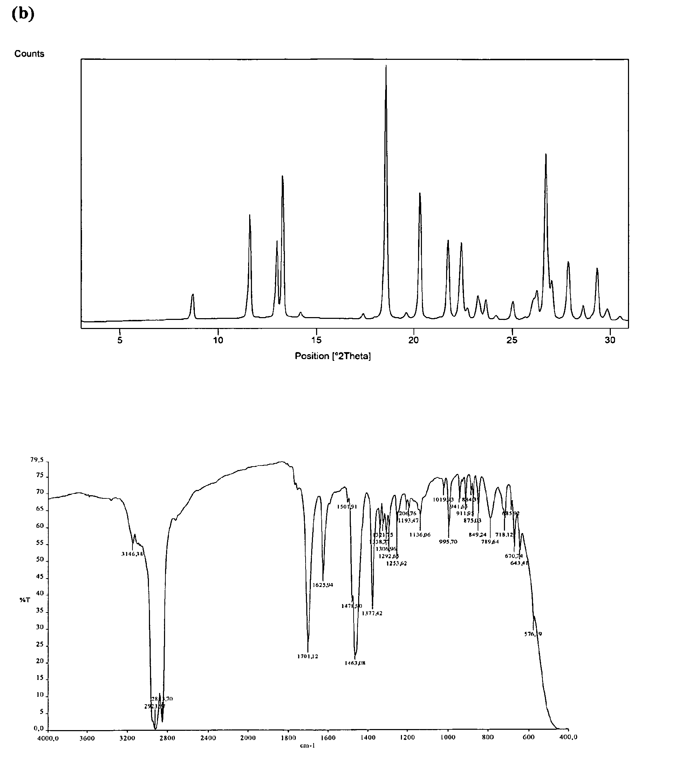

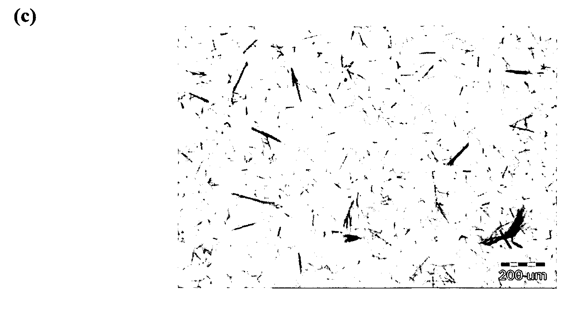

Figure 1 shows an X-ray powder diffraction spectrum of 6-Chloro-5-(2-Chloroethyl)-1,3-Dihydro-2H-Indol-2-One (INT 1).

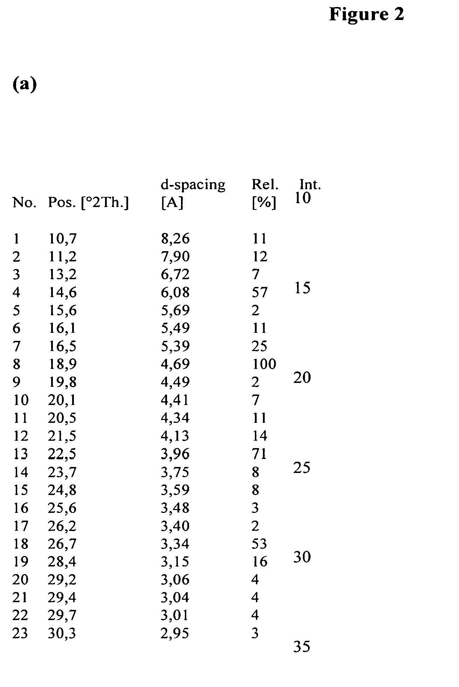

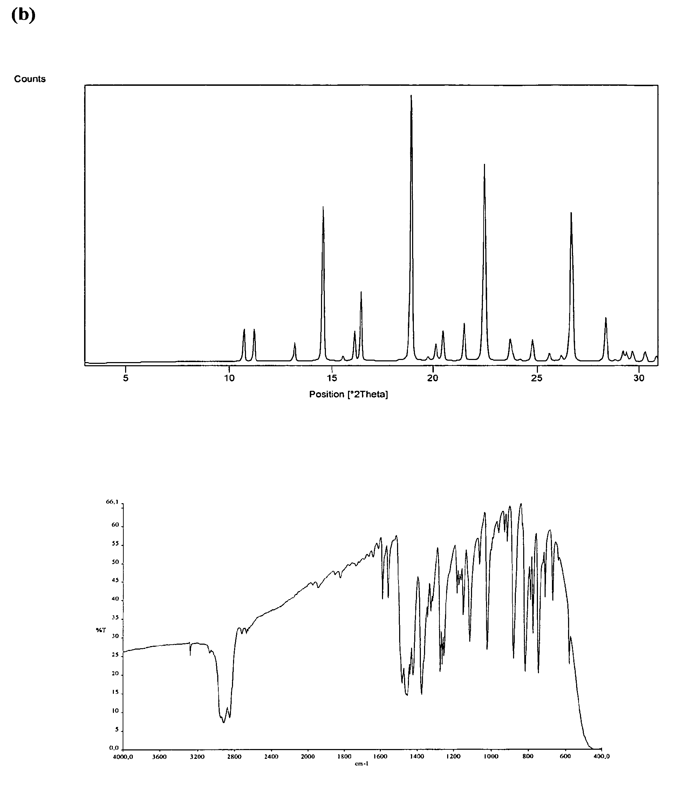

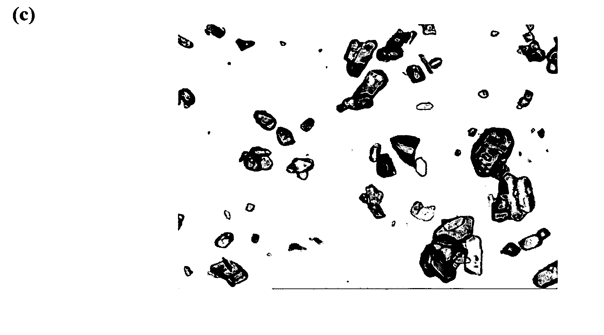

Figure 2 shows an X-ray powder diffraction spectrum of (3-(1-piperazinyl)1,2-benzisothiazole) (INT 2).

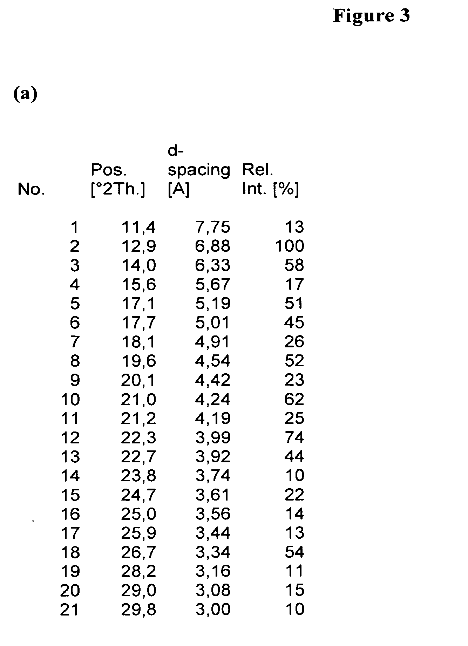

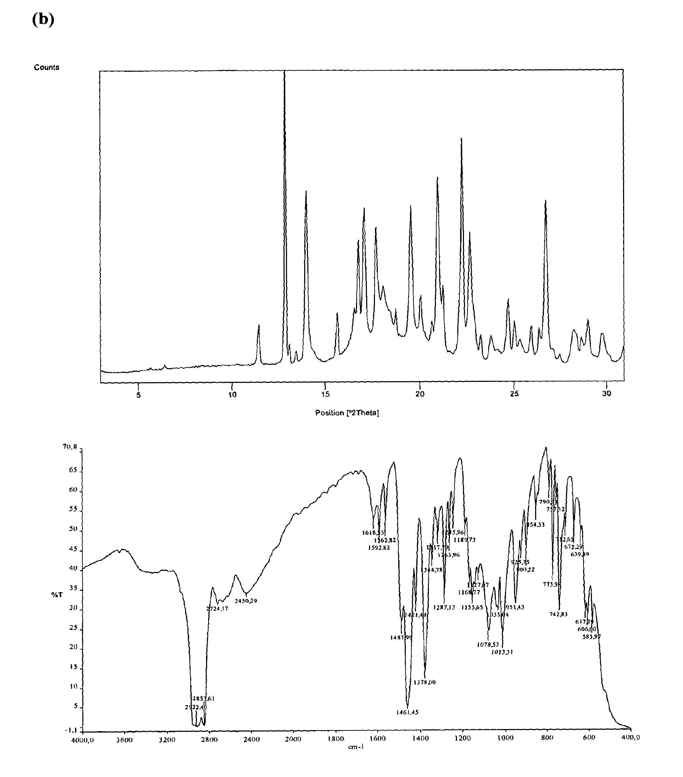

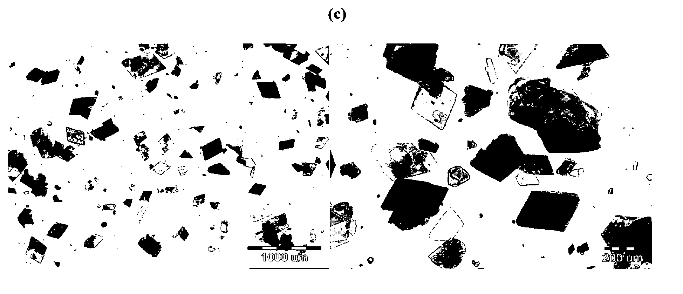

Figure 3 shows an X-ray powder diffraction spectrum of (3-(1-piperazinyl)1,2-benzisothiazole) sulphate hydrate (INT 2 sulphate hydrate).
Figure 4 shows an X-ray powder diffraction spectrum of 5- [2- [4-(1,2-benzisothiazol-3-yl) -1-piperazinyl] ethyl] -6-chloro-1, 3- dihydro-2H-indol-2-one hydrogensulphate dihydrate (Ziprasidone hydrogensulphate dihydrate).
Figure 5 shows an X-ray powder diffraction spectrum of 5-[2-[4-(1,2-benzisothiazol-3-yl)-1-piperazinyl] ethyl] -6-chloro-1, 3- dihydro-2H-indol-2-one hydrogensulphate anhydrous.

### Detailed description of the invention

According to a first embodiment of the present invention a compound with formula I, a Ziprasidone hydrogensulphate, is provided having the general formula:

[Ziprasidone * H]⁺ [X⁻] * ZH₂O

wherein Ziprasidone is a compound of the formula 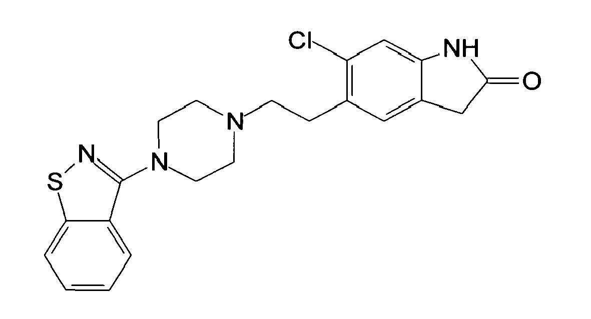 H is hydrogen
X is HSO₄⁻; and
Z is 0 to 30, preferably 0 or 2.

Z≤0.28 designates hereby the essential water free or anhydrous compound I with an approximate water content below 1 % per weight.

Preferably, the compound is either anhydrous [Ziprasidone-H]⁺ * HSO₄⁻ or [Ziprasidone-H]⁺ * HSO₄⁻ * 2H₂O.

According to an embodiment, the present compound has a particle size in the range of about 5 µm to about 300 µm, preferably in the range of about 10 µm to about 150 µm and more preferably in the range of about 20 µm to about 80 µm.

The average particle size or particle size referring to the volume mean diameter of particles of the present compounds may be determined by any suitable method known to the skilled person. Examples for suitable methods are based for example on sedimentation, by laser light scattering laser diffraction methods or electrical differential mobility analysis. Preferably, laser light scattering is employed.

In another embodiment, the compound according to the present invention has a solubility in the range of about 50 mg/l to about 800 mg/l, preferably in the range of about 100 mg/l to about 500 mg/l and more preferably in the range of about 250 mg/l to about 400 mg/l.

Different methods for detecting the water content known to the skilled person may be used such as the method according to Karl Fischer, which is described inter alia in European Pharmacopeia 5th edition 2006; Method 2.5.12, which document is incorporated herein by way of reference.

Both small average particle size and high solubility favour a high bioavailability - representing a measure of the rate and extent of a therapeutically active drug that reaches the systemic circulation and which is available at the site of action - of the present compound.

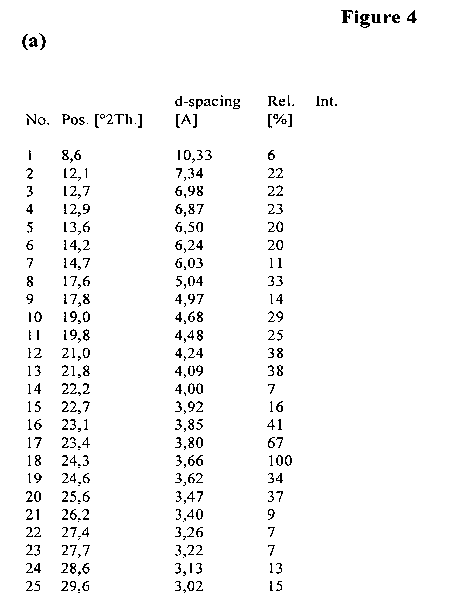

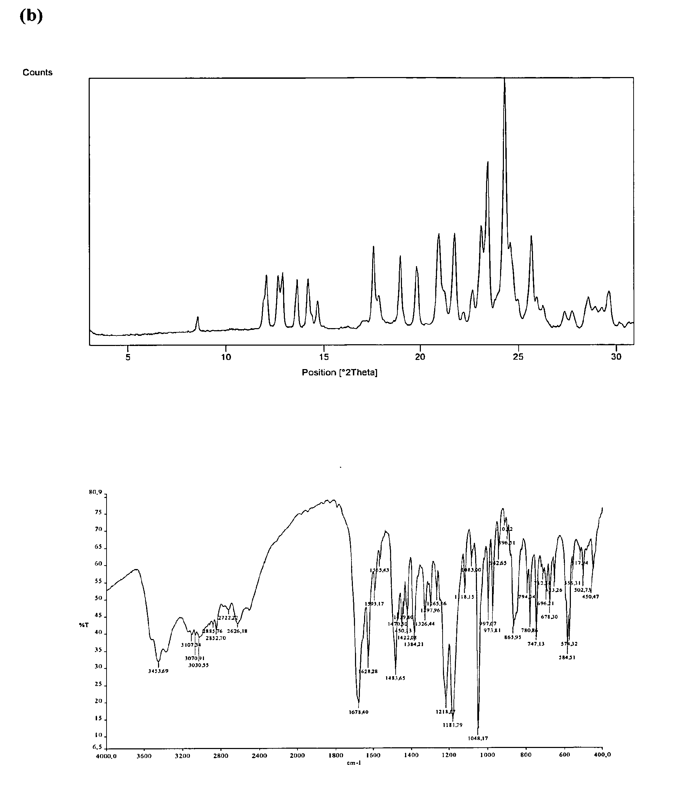

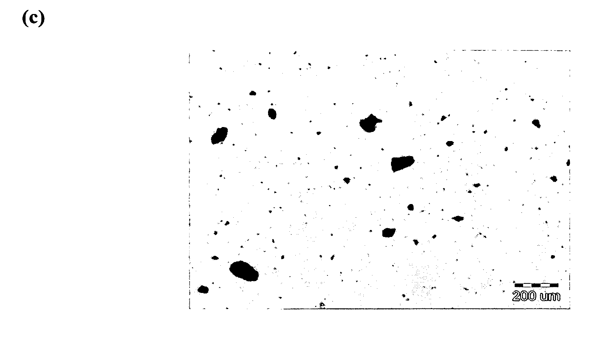

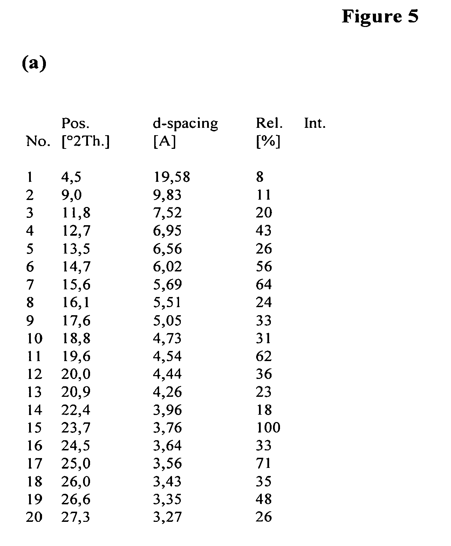

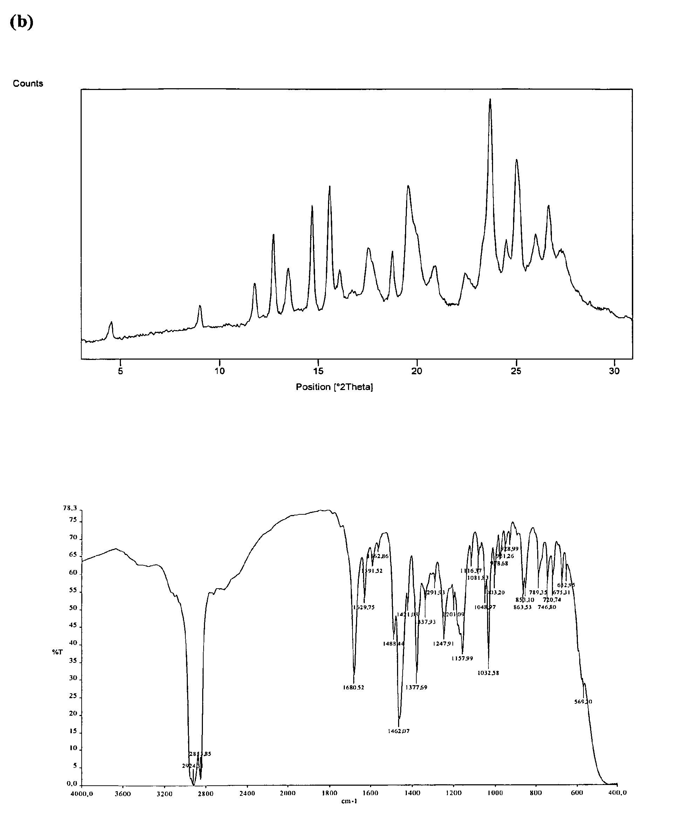

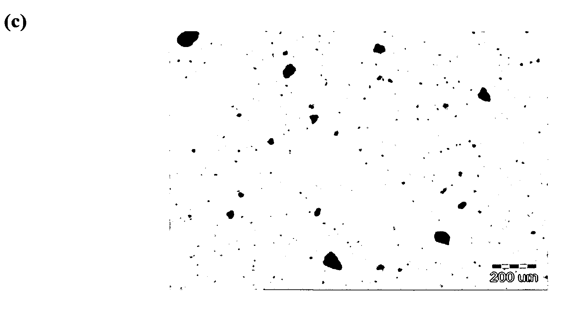

According to another embodiment, the present compounds are characterized by a X-ray powder diffraction pattern exhibiting characteristic diffractions at 8,6; 12,1; 13,6; 17,6; 19,8; 23,4 in 24,3°± 0,2°2Theta (Form I) or 4,5; 9,0; 15,6; 23,7; 25,0 in 26,6°± 0,2°2Theta (Form II). Preferably, the Form I and II are characterized by a X-ray powder diffraction pattern as outlined in Figure 4 and Figure 5, respectively. X-ray powder diffraction patterns are particularly suitable to describe polymorphic forms.

The discovery of new polymorphic forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

According to another preferred embodiment of the present invention, a process for the preparation of compounds according to the present invention is provided. The process comprises the steps of (i) providing anhydrous Ziprasidone base, (ii) reacting anhydrous Ziprasidone base with defined amounts of both water and sulfuric acid, (iii) optionally cooling and/or drying, and (iv) obtaining compound of formula I having a defined water content, characterized by specific crystal structures.

Ziprasidone base or crude Ziprasidone may be prepared e.g. by reacting 6-Chloro-5-(2-chloroethyl)-1,3-dihydro-2H-indol-2-one (INT 1) and (3-(1-piperazinyl)1,2-benzisothiazole) hydrogen sulphate (INT 2) as outlined in the examples. Alternatively, any other reaction(s) yielding e.g. crude Ziprasidone hydrochloride, which are for example outlined in EP 0 281 309, EP 0 584 903, EP 1 029 861, WO2006/034964, WO2006/034965, WO2006/011157, WO2006/047893, WO2005/085240, WO2005/0401650, US2006/0089502, may be used. Such compounds may be in turn converted to Ziprasidone sulphates by methods known to the skilled person.

The respective anhydrous compound I, characterized by Z≤0.28, may be obtained by standard drying procedures for removing solvents. Examples for suitable drying procedures comprise application of low pressure or desiccator containing one or more chemicals suitable for removing solvent by e.g. binding to them.

In order to obtain the desired product, i.e. Ziprasidone sulphate with a defined water content, the anhydrous compound is reacted with defined amounts of sulfuric acid and water. Alternatively, an excess of water may be employed followed by a drying step, e.g. by means of an air dryer or any other drying method as outlined above, to the desired extend (as determined e.g. by the method according to Karl Fischer).

It should be appreciated that sulfuric acid of any water content or essential water free sulfuric acid or even oleum may be used for the above mentioned conversion to the desired compound. In such cases the water content of the sulfuric acid may be considered and incorporated in calculating the ratio of sulfuric acid and water used.

Any kind of ratio of anhydrous Ziprasidone base, sulfuric acid and water may be used for performing the reaction.

Duration and temperature of the reaction may be chosen according to common knowledge. Preferably the reaction is conduced for 1-6 hours at temperatures in the range of 20-80°C. More preferably, the reaction is conduced first at ambient temperature, i.e. in a range between 20 and 25°C, for 10-120 minutes and than heated to 50-70°C for other 2-5 hours.

After performing the reaction the mixture obtained may be optionally cooled, filtered and/or dried.

According to another embodiment, anhydrous Ziprasidone base may be provided by reacting 6-Chloro-5-(2-chloroethyl)-1,3-dihydro-2H-indol-2-one and 3-(1-piperazinyl)1,2-benzisothiazole in presence of an alkaline compound and an organic solvent which is preferably selected from the group of ionic liquids. Examples for suitable alkaline compounds comprise Na₂CO₃ K₂CO₃ and for ionic liquids comprise 1-Ethyl-3-methylimidazolium hydrogen sulphate, 1-Ethyl-3-methylimidazolium methyl sulphate, 1-butyl-3-methylimidazolium tetrafluoroborate and 1-butyl-3-methylimidazolium bromide. Also mixtures of one or more ionic liquids with water can be used as well as mixtures thereof with water.

The reaction may be also carried out in the presence of sodium iodide and/or potassium iodide acting as a catalyst.

Preferably, Na₂CO₃ and 1-Ethyl-3-methylimidazolium methyl sulphate are employed. Respective amounts of the compounds, reaction temperature and duration are known to the skilled person.

Preferably, the reaction is performed at a temperature ranging from room temperature to reflux temperature of the solvent applied, more preferably at the temperature selected from 80°C to 120°C. Preferably, the reaction is kept at the selected temperature for 3 to 80 h, more preferably for 10 to 30 h. The term "room temperature" as used herein refers to a temperature of about 22°C.

In an another embodiment, the Ziprasidone base could be prepared by reduction of 5-[2-[4-(1,2-benzisothiazol-3-yl)-1-piperazinyl]-1-oxoethyl]-6-chloro-1,3-dihydro-2H-indol-2-one ("oxoethyl-Ziprasidone") or addition salts thereof.

After the completion of the reaction the reaction mixture is preferably cooled to room temperature and the product is precipitated by adding water or organic solvent or mixtures thereof. In a preferred embodiment organic solvents are selected from C₁-C₅ alcohol, acetonitrile or tetrahydrofuran, acetic acid, acetone and/or ethers. More preferably alcohols are selected from methanol, ethanol and isopropanol and ether is selected from isopropyl ether.

In a preferred embodiment, the Ziprasidone base is further purified either by hot maceration or precipitation in the above mentioned solvents. Both purification methods are well known to the skilled person.

Ziprasidone sulphate salts may be prepared by mixing a Ziprasidone base or a mixture of Ziprasidone base and Ziprasidone salt with an organic or inorganic base in the above mentioned solvents or mixtures thereof, at a temperature ranging from the room to reflux temperature of the solvent applied, preferably at a temperature ranging between 50°C to 60 °C.

The solution can be filtered and then cooled to a temperature between -10 and 30°C, preferably to room temperature.

The precipitate is filtered and the product is dried preferably at a temperature between 10°C and 60°C, more preferably between 40 °C and 50°C. Drying may be achieved e.g. via application of heat, preferably carried out under ambient or reduced pressure or via contacting a Ziprasidone salt with humid air in a fluidized bed drier, wherein the atmosphere in the fluidized bed drier is at least 15% humidity. Preferably, air drying in a commercially available humid air dryer is applied.

According to a preferred embodiment, the present compounds are used for the preparation of a pharmaceutical composition for the prevention and/or treatment of psychotic states and preferably schizophrenia and anxiety diseases.

Suitable pharmaceutical compositions comprise preliminary enteral and parenteral Pharmaceutical compositions employing respective carriers in different amounts.

The pharmaceutical compositions may be in a solid dosage form. Exemplary solid dosage forms include tablets, capsules, sachets, lozenges, powders, pills or granules. The solid dosage form may be, for example, immediate release dosage form, a fast melt dosage form, controlled release dosage form, lyophilized dosage form, delayed release dosage form, extended release dosage form, pulsatile release dosage form, mixed immediate release and controlled release dosage form, or a combination thereof. A solid dose tablet formulation is preferred. The solid dosage form is preferably an immediate release dosage form offering advantages regarding the bioavailability of the active compound.

The dosage form may produced in accordance with usual techniques in which the active ingredient or substance and one or more are mixed and granulated by adding solvent in a low or high shear mixer or by fluidized bed granulator. The granulate is dried, for example in a fluidized bed dryer. The dried granulate is sized. The sizing of the micromatrix particles may be performed by using an oscillating granulator, comminuting mill or any other conventional method. The sieve used for the sizing may have openings from 0.25 mm to 5 mm. Alternatively, the core particles can be made by extrusion, spheronization, melt granulation or by roller compaction. The core particles may be coated by a solution of one or more release controlling agents by any known method, including spray application. Spraying can be carried out using a fluidized bed coater (preferably Wurster coating), or in a pan coating system. Alternatively the coating of the core particles with one or more rate controlling agents can be done by hot melt process using a granulator or fluidized bed coater (preferably Wurster coating), or in a pan coating system. The compression of micro tablets is carried out on usual compression machines (e. g. machines by Manesty, Cadmach or Kilian). The micro tablets can be made of various sizes and shapes like round, oval, oblong, capsule shaped, triangular, square, etc. The preferred shape of the micro tablet is round, biconvex and the preferred diameter of the micro tablet is 1.5 mm to 9.5 mm.

The micro tablets may be coated by a solution of one or more release controlling agents by any known method, including spray application. Spraying can be carried out using a fluidized bed coated (preferably Wurster coating), or in a pan coating system.

The present composition may by also present in a particular dosage form for further improving the bioavailability of the present compound. The bioavailability of orally ingested drugs is determined by factors, which include the nature of the molecule, its stability, and the formulation administered - and in the patient - such as a reduced intestinal surface area as a result of colic disease or intestinal resection and whether or not the drug is taken with a meal. Factors influencing the bioavailability may include, but are not limited to a poor absorption from the gastrointestinal tract, hepatic first-pass effect and degradation of the drug prior to reaching system circulation.

For improving bioavailability of the present compound, e.g. the particle size fractions may be adapted by further crushing. Preferable particle size after crushing is d₉₀, i.e. the average particle diameter of 90% of the particles, less than 30 µm, more preferably less than 20 µm, most preferably less than 10 µm.

According to another embodiment, the present pharmaceutical composition may contain in addition to the present compounds one or more diluents, binding agents, disintegrants, lubricants, sweeteners, glidants, flavourings, colouring agents and other excipients, depending on the dosage form desired.

Suitable diluents include pharmaceutically acceptable fillers such as lactose, microcrystalline cellulose, dibasic calcium phosphate, saccharides and/or mixtures of the foregoing. Examples of diluents include microcrystalline cellulose, such as Avicel PH 101^{®} and Avicel^{®} PH 102; lactose such as lactose monohydrate, lactose anhydrous and Pharmatose^{®} DCL 21; dibasic calcium phosphate such as Emcompress^{®}; mannitol, starch, sorbitol, sucrose and glucose. The most preferred are microcrystalline cellulose and lactose.

Binding agents are preferably selected from polyvinylpyrolidone, starch grades (pre-gellatinized or plain), cellulose derivatives such as hydroxypropylmethylcellulose (HPMC), hydroxypropylcellulose (HPC) and carboxymethylcellulose (CMC) and their salts and gelatine, the most preferred is HPMC.

Suitable disintegrants include croscarmellose sodium, crospovidone, sodium starch glycolate, corn starch, potato starch, maize starch and modified starches calcium silicates, low substituted hydroxypropylcellulose and the like. Most preferred is croscarmellose sodium.

Lubricants are preferably selected from the group consisted of magnesium stearate, magnesium lauryl sulphate and sodium stearyl fumarate, sucrose esters or fatty acid, polyethylene glycol, stearic acid and the like.

Sweeteners are preferably selected from the group consisting of aspartame, saccharin sodium, dipotassium glycyrrhizinate, aspartame, stevia, thaumatin, and the like.

Glidants are preferably selected from the group consisting of silicon dioxide, talc and aluminium silicate.

As flavourings, colouring agents, or opacifying agents and pigments any suitable compound known to the skilled person may be used.

Compositions suitable for parenteral administration comprise sterile aqueous and non-aqueous injection solutions of the active compound, which preparations are preferably isotonic with the blood of the intended recipient.

These preparations may contain anti-oxidants, buffers, bacteriostats and solutes, which render the compositions isotonic with the blood of the intended recipient. Aqueous and non-aqueous sterile suspensions may include suspending agents and thickening agents. The compositions may be presented in unit-dose or multi-dose containers, for example sealed ampoules and vials, and may be stored in a freeze-dried or a lyophilized condition requiring only the addition of the sterile liquid carrier, for example, saline or water-for-injection immediately prior to use. Extemporaneous injection solutions and suspensions may be prepared from sterile powders, granules and tablets of the kind previously described.

The concentration of active compounds in the present composition will depend on e.g. absorption, inactivation, excretion rates, the dosage schedule and amount administered as well as other factors known to those of skilled in the art. Other ingredients to produce e.g. an enteral applicable composition, such as a tablet or capsule or another suitable form, are also well known to the skilled person and may be formulated depending on the desired consistency and other properties envisaged.

The following examples illustrate the invention without limiting it thereto.

### Examples

The compounds according to the present invention were characterized with regard to their meltings points by means of a Koffler melting point apparatus, IR spectra taken on a Paragon 100 Perkin-Elmer FT-IR spectrometer (performed on a FT-IR System SPECTRUM GX Perkin Elmer [4000-400] cm⁻¹ at a resolution 4 cm⁻¹, preparing KBr tbl.) and X-ray powder diffraction patterns (obtained by a Phillips PW3040/60 X'Pert PRO diffractometer using CuK_{α} radiation). Water contents of the respective compounds have been tested by the method according to Karl Fischer. Images of particles were taken on a Microscope Olympus BX 50 equipped with Olympus camera DP70.

A high resolution HPLC method is used to determine an amount and purity of INT1, INT 2, oxoethyl-Ziprasidone and Ziprasidone base. The tests are carried out in Zorbax Eclipse XDB-C18, 1,8 µm, 50 x 4,6 mm column and X-bridge C18, 2.5 µm, 75 x 4.6 mm column. The mobile phase is gradient of 0.01 M diammonium hydrogenphosphate, pH 3 and acetonitrile /methanol (20/80 ratio). The chromatograph is equipped with a UV detector set at 230 nm, the flow rate is 1.0 ml per minute at 40°C.

The average particle size is determined by laser light scattering using a Malvern-Mastersizer Apparatus MS 2000. The particles to be subjected to the particle size measurement are first suspended in isoparafinic oil (Isopar^{®}) and then subjected to a size determination in a Malvern Mastersizer MS 2000 instrument. Usually, 100-800 mg of substance is dispersed in 5-8 ml of vegetable oil. According to manufacturer's information, the Malvern Mastersizers allow for a measurement of particle size distributions in the range of 20 nm to 2000 µm, with a precision of better than 0.5%. Particle sizes are determined by measuring the angular distribution of laser light scattered by a homogeneous suspension of particles. The size distribution is determined from the light scattering data using the theory of light scattering developed by Gustav Mie.

### Example 1

### Preparation of (3-(1-piperazinyl)1,2-benzisothiazole) sulphate hydrate (INT 2 sulphate)

An amount of 1 g of (3-(1-piperazinyl)1,2-benzisothiazole), 10 ml of demineralized water and 1.9 g of H₂SO₄ (INT2 : H₂SO₄ ratio is 1:5) were mixed at room temperature overnight. After the completion of the process clear solution became white. The product was filtered and dried for 2 hours at 30 °C. An amount of 0.3 of white precipitate of (3-(1-piperazinyl)-1,2-benzisothiazole) hydrogen sulphate hydrate is obtained. The water content, determined according to Karl Fischer was 2.5 %.

### Example 2

### Preparation of (3-(1-piperazinyl)1,2-benzisothiazole) sulphate hydrate (INT 2 sulphate)

An amount of 50 g of (3-(1-piperazinyl)-1,2-benzisothiazole), 500 ml of demineralized water and 33,5 g of H2SO4 (INT 2 : H2SO4 ratio is 1 : 1,5) were mixed at room temperature for 47 hour. After the completion of process clear solution became white. The product was filtered, washed with 125 ml demineralized water and dried for 2 hours at 30 C. An amount of 42,6 g of white precipitate of (3-(1-piperazinyl)-1,2-benzisothiazole) sulphate hydrate is obtained.

### Example 3

### Preparation of 5-[2-[4-(1,2-benzisothiazol-3-yl)-1-piperazinyl]ethyl]-6-chloro-1,3-dihydro-2H-indol-2-one (Ziprasidone base)

In a round bottom flask 1 g of 6-Chloro-5-(2-Chloroethyl)-1,3-Dihydro-2H-Indol-2-One (INT 1), 1.1 g of (3-(1-piperazinyl)1,2-benzisothiazole), 1.46 g of Na2C03 and 8 ml of 1-ethyl-3-methylimidazolium methyl sulphate are placed, heated to 100 °C and stirred for 24 hours. After the completion of the reaction the presence of 5- [2- [4- (1, 2- benzisothiazol-3-yl)-1-piperazinyl] ethyl] -6-chloro-1, 3- dihydro-2H-indol-2-one is confirmed by HPLC. The reaction mixture is cooled to room temperature and 30 ml of methanol is added to precipitate the product. Reaction mixture was stirred for about 1 h and filtered. An amount of 1.8 g of wet crude Ziprasidone base was obtained (Purity 88.0 % was determined by HPLC). A whole sample was further placed in a 50 ml round bottom flask with 20 ml of demineralised water and heated at the 79°C for 30 minutes, filtrated and cooled to room temperature. A 2.65 g of precipitated wet Ziprasidone base was dried in a vacuum drier for 1 h at the temperature 50 °C and overnight at the room temperature. An amount of 1.03 g of Ziprasidone base was obtained.

### Example 4

### Preparation of 5-[2-[4-(1,2-benzisothiazol-3-yl)-1-piperazinyl]ethyl]-6-chloro-1,3-dihydro-2H-indol-2-one (Ziprasidone base)

Example 2 was repeated with the exception that 1-butyl-3-methylimidazolium tetrafluoroborate is used instead of 1-ethyl-3-methylimidazolium methyl sulphate. After the completion of the reaction the presence of 5- [2-[4-(1,2-benzisothiazol-3-yl)-1-piperazinyl]ethyl] -6-chloro-1,3-dihydro-2H-indol-2-one is confirmed by HPLC.

### Example 5

### Preparationof5- [2- [4- (1, 2- benzisothiazol-3-yl) -1-piperazinyl] ethyl] -6-chloro-1, 3-dihydro-2H-indol-2-one (Ziprasidone base)

Example 2 was repeated with the exception that 10 ml of 1-butyl-3-methylimidazolium bromide is used instead of 8 ml of 1-ethyl-3-methylimidazolium methyl sulphate. After the completion of the reaction the presence of 5-[2-[4-(1,2-benzisothiazol-3-yl)-1-piperazinyl] ethyl] -6-chloro-1, 3- dihydro-2H-indol-2-one is confirmed by HPLC.

### Example 6

### Preparation of 5- [2-,[4-(1,2-benzisothiazol-3-yl)-1-piperazinyl]ethyl]-6-chloro-1,3-di-hydro-2H-indol-2-one hydrogensulphate dihydrate (Ziprasidone hydrogensulphate dihydrate)

In a round bottom flask 2 g of anhydrous (containing less than 1 % water defined by Karl-Fischer) 5-[2-[4-(1,2-benzisothiazol-3-yl)-1-piperazinyl]ethyl]-6-chloro-1,3-dihydro-2H-indol-2-one (Ziprasidon base), 20 ml demineralised water and 0.95 g of sulphuric acid are placed and mixed at the room temperature 15 to 60 minutes. A homogenous mixture is heated to 60 °C and intensively mixed for about 3 hours. The mixture is then cooled to room temperature, filtrated and rinsed with demineralsed water. Obtained product is dried in an air drier for about 2 hours at the 30°C until a constant weight is obtained. 2.46 g of the product is obtained. Water content is 7.1 % according to Karl-Fischer.

Solubility of the obtained Ziprasidone hydrogensulphate dihydrate is 0.36 mg/ml in water (pH 6.8) + 2 % SDS at room temperature. A two-week stability tests (40°C, humidity 75% and 50°C) show no degradation of the initial product ending with determined HPLC purity 99.7 % and 99.7 % of Ziprasidone, respectively.

### Example 7

### Preparation of 5-[2-[4-(1,2-benzisothiazol-3-yl)-1-piperazinyl]ethyl]-6-chloro-1,3-dihydro-2H-indol-2-one hydrogensulphate dihydrate (Ziprasidone hydrogensulphate dihydrate)

In a round bottom flask 23 g of anhydrous (containing less than 2 % water defined by Karl-Fischer) 5-[2-[4-(1,2-benzisothiazol-3-yl)-1-piperazinyl] ethyl]-6-chloro-1,3-dihydro-2H-indol-2-one (Ziprasidone base), 230 ml demineralised water and 11g of sulphuric acid are placed and mixed at the room temperature 15 to 60 minutes. A homogenous mixture is heated to 60 °C and intensively mixed for about 3 hours. The mixture is then cooled to room temperature, filtrated and rinsed with demineralised water. Obtained product is dried in an air drier for about 2 hours at the 30°C until a constant weight is obtained. 29.9 g of the product is obtained. Water content is 6.8 % according to Karl-Fischer.

### Example 8

### Preparation of 5-[2-[4-(1,2-benzisothiazol-3-yl)-1-piperazinyl]ethyl]-6-chloro-1,3-dihydro-2H-indol-2-one hydrogensulphate dihydrate (Ziprasidone hydrogensulphate dihydrate)

In a round bottom flask 7.5 g of anhydrous (containing less than 1 % water defined by Karl-Fischer)5-[2-[4-(1,2-benzisothiazol-3-yl)-1-piperazinyl] ethyl] -6-chloro-1, 3- dihydro-2H-indol-2-one (Ziprasidone base), 75 ml demineralised water and 1.95 g of sulphuric acid are placed and mixed at the room temperature 15 to 60 minutes. A homogenous mixture is heated to 60 °C and intensively mixed for about 3 hours. The mixture is then cooled to room temperature, filtrated and rinsed with demineralised water. Obtained product is dried in an air drier for about 2 hours at the 30°C until a constant weight is obtained. 10.1 g of the product is obtained. Water content is 6.9 % according to Karl-Fischer.

### Example 9

### Preparation of 5-[2-[4-(1,2-benzisothiazol-3-yl)-1-piperazinyl]ethyl]-6-chloro-1,3- dihydro-2H-indol-2-one hydrogensulphate dihydrate (Ziprasidone hydrogensulphate anhydrous)

An amount of 300 mg of Ziprasidone hydrogensulphate dihydrate as obtained in Example 6 was dried in for 12 h at the temperature of 100 °C in an air drier. The water content of the obtained ziprasidone hydrogen sulphate anhydrous is less than 0.5 % according to Karl-Fischer.

### Example 10

### Preparation of 5-[2-[4-(1,2-benzisothiazol-3-yl)-1-piperazinyl]ethyl]-6-chloro-1,3-dihydro-2H-indol-2-one hydrogensulphate dihydrate (Ziprasidone hydrogensulphate anhydrous)

An amount of 1 g of ziprazidone hydrogensulphate dehydrate as obtained in example 6 was dried in for 12 h at the temperature in a vacuum drier. The water content of the obtained ziprazidone hydrogensulphate anhydrous is less than 0,5 % according to Karl Fischer.

### Example 11

An amount of 20 mg of zipasidone hydrogensulphate dihydrate was exposed to a water-sorption analysis (Surface Measurement Systems Ltd., London, UK; two complete 11-step circles from 0 % RH to 95 % RH at the temperature 25°C and nitrogen flow rate 200 ml/min; minimal retention time at the step when dm/dt < 0.002% is 10 minutes and maximal 360 minutes) and compared to the same test of Ziprasidone hydrochloride monohydrate.

Ziprasidone hydrogensulphate salt is more stable to hydratation with the regard to Ziprasidone hydrochloride salt. Zipasidone hydrogensulphate dihydrate does not release crystal water at the 0 % RH at the performed test, comparing to Ziprasidone hydrochloride monohydrate which release crystal water at 0 % RH. Furthermore, water sorption of Ziprasidone hydrochloride started at 30 % RH, whereas water sorption of zipasidone hydrogensulphate started at 50 % RH.

### Example 12

### Formulation

All the compositions contain 35.26 Wt. % of Ziprasidone hydrogensulphate dihydrate, which is equivalent to 26.66 Wt. % of Ziprasidone free base. Particle size of the Ziprasidone hydrogensulphate dihydrate is 26 µm.

### Example 12. A

| Ingredients | Wt. % Composition |
|---|---|
| Ziprasidone hydrogensulphate dihydrate | 35.26 |
| Lactose Monohydrate | 54.48 |
| Pregelatinized Starch | 9.12 |
| Magnesium Stearate (I) | 0.91 |
| Magnesium Stearate (II) | 0.23 |
| Total | 100.0 |

Ziprasidone hydrogensulphate dihydrate capsules were manufactured by direct fill process. All the components were sieved and homogenized. The resulting blend was filled into size 2 hard gelatin capsules, each containing 300 mg of the composition.

The formulation can also be prepared using dry granulation process. In this case the Ziprasidone hydrogensulphate dihydrate was combined with lactose monohydrate, pregelatinized starch and magnesium stearate (I). The powder mixture was sieved, dry compacted and the agglomerates were milled. Then the magnesium stearate (II) was added and the resulting was filled into size 2 hard gelatine capsules, each containing 300 mg of the composition.

Another useful process of preparation is wet granulation. In the scope of this method, Ziprasidone hydrogensulphate dihydrate, lactose monohydrate and pregelatinized starch were sieved and blended. The powder mixture was granulated by adding purified water, while kneading the mixture. Drying of wet granulate was performed in the oven at 50°C for 4 hours. The magnesium stearate was added and the resulting blend was filled into size 2 hard gelatine capsules, each containing 300 mg of the composition.

### Example 12. B

| Ingredients | Wt. % Composition |
|---|---|
| Ziprasidone hydrogensulphate dihydrate | 35.26 |
| Lactose Monohydrate | 49.48 |
| Pregelatinized Starch | 9.12 |
| Polyethylene glycol 6000 | 5.0 |
| Magnesium Stearate | 1.14 |
| Total | 100.0 |

The pharmaceutical composition was prepared by wet granulation process at the batch size of 0.5 kg. Ziprasidone hydrogensulphate dihydrate, lactose monohydrate, pregelatinized starch and polyethylene glycol were sieved and blended. Granulation was performed by adding purified water, while kneading the mixture. The wet granulate was dried in the oven at 50°C for 4 hours. After magnesium stearate was added, the resulting blend was filled into size 2 hard gelatine capsules, each containing 300 mg of the composition.

### Example 12. C

| Ingredients | Wt. % Composition |
|---|---|
| Ziprasidone hydrogensulphate dihydrate | 35.26 |
| Lactose Monohydrate | 59.19 |
| Sodium starch glycolate | 3.97 |
| Sodium lauryl sulphate | 0.39 |
| Amorphous silica | 0.39 |
| Magnesium Stearate | 0.80 |
| Total | 100.0 |

Ziprasidone hydrogensulphate dihydrate capsules were manufactured by direct fill process. All the components were sieved and homogenized. The resulting blend was filled into size 2 hard gelatine capsules, each containing 300 mg of the composition.

## Claims

1. Compound of formula I:
[Ziprasidone * H]⁺[X] * Z H₂O
wherein
Ziprasidone is a compound of the formula 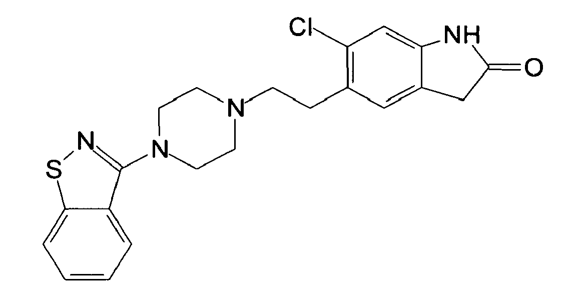 H is hydrogen
X is HSO₄⁻ ; and
Z is 0 to 30.

2. The compound according to any of the proceeding claims, wherein Z is 0 or 2.

3. The compound according to any of the proceeding claims, wherein said compound has a particle size in the range of about 5 µm to about 300 µm.

4. The compound according to any of the proceeding claims, wherein said compound has a solubility in the range of about 50 mg/l to about 800 mg/l.

5. The compound according to claim 1, **characterized by** a X-ray powder diffraction pattern exhibiting characteristic diffraction angles at 8.6; 12.1; 13.6; 17.6; 19.8; 23.4 and 24.3°± 0.2° Theta.

6. The compound according to claim 1, **characterized by** a X-ray powder diffraction pattern exhibiting characteristic diffraction angles at 4.5; 9.0; 15.6; 23.7; 25.0 and 26.6°± 0.2°2 Theta.

7. A process for the preparation of a compound of formula I, comprising the steps of:
- providing anhydrous Ziprasidone base;
- reacting with water and sulfuric acid;
- optionally cooling and/or drying; and
- obtaining compound of formula I.

8. The process according to claim 7, wherein anhydrous Ziprasidone base is provided by reacting 6-Chloro-5-(2-Chloroethyl)-1,3-Dihydro-2H-Indol-2-One and 3-(1-piperazinyl)-1,2-benzisothiazole in presence of an alkaline compound and an organic solvent, optionally mixed with water.

9. The process according to claim 8, wherein the alkaline compound is Na₂CO₃.

10. The process according to claim 8, wherein the organic solvent is selected from the group consisting of 1-Ethyl-3-methylimidazolium methyl sulphate, 1-Ethyl-3-methylimidazolium hydrogen sulphate 1-butyl-3-methylimidazolium tetrafluoroborate and 1-butyl-3-methylimidazolium bromide.

11. Use of a compound according to any of the claims 1-7 for the preparation of a pharmaceutical composition for the prevention and/or treatment of psychotic states.

12. The use according to claim 11, wherein the psychotic states include schizophrenia and anxiety diseases.

13. (3-(1-piperazinyl)1,2-benzisothiazole) sulphate hydrate **characterized by** a X-ray powder diffraction pattern exhibiting characteristic diffraction angles at 12.9; 14.0; 17.1; 19.6; 21.0; 22.3 and 26.7°± 0,2° Theta.
